# EUROPEAN PATENT SPECIFICATION

(11) **EP 1 945 174 B1**
(45) Date of publication and mention of the grant of the patent: **13.08.2014**
(21) Application number: 06827765.6
(22) Date of filing: 09.11.2006
(51) Int. Cl.: A61K 6/00

(54) **DENTURE ADHESIVE ARTICLES**
GEBISS-KLEBEARTIKEL
ARTICLES SERVANT DE CIMENT POUR PROTHÈSE DENTAIRE

(30) Priority: 09.11.2005 US 735243 P; 09.11.2005 US 735088 P; 09.11.2005 US 735136 P; 09.11.2005 US 735135 P; 09.11.2005 US 734874 P; 20.01.2006 US 760528 P; 20.01.2006 US 760660 P; 20.01.2006 US 760516 P; 20.01.2006 US 760526 P; 20.01.2006 US 760527 P; 20.01.2006 US 760711 P; 31.10.2006 US 590145; 31.10.2006 US 590232; 31.10.2006 US 590224; 31.10.2006 US 590233; 31.10.2006 US 590111; 31.10.2006 US 590225; 31.10.2006 US 590191; 31.10.2006 US 590231
(43) Date of publication of application: 23.07.2008
(73) Proprietor: The Procter & Gamble Company, Cincinnati, OH 45202 (US)
(72) Inventor: RAJAIAH, Jayanth, Loveland, OH 45140 (US); CULBERTSON, Robert, Hargitt, Cincinnati, OH 45208-27212 (US); CERDA, Luisa, Navarro, Cincinnati, OH 45242 (US); LEONARD, Robert, Scott, Fairfield, OH 45014 (US); CAPPEL, Barbara, Ann, Monroe, OH 45050 (US)
(74) Representative: Joos, Uta Susanne
(86) International application number: PCT/US2006/044028
(87) International publication number: WO 2007/056605

(56) References cited:
- WO-A-01/15657
- WO-A-01/41710
- WO-A-02/30317
- WO-A-96/25910
- WO-A-2005/081935
- US-A1- 2003 108 489

## Description

### TECHNICAL FIELD

In one embodiment this invention relates to denture adhesive articles comprising dry tack and in particular to improved denture adhesive articles comprising a mixture of a denture adhesive component with a water insoluble component selected from polyethylene, microcrystalline wax, elastomers and mixtures thereof, and a non-adhesive self-supporting layer.

### BACKGROUND OF THE INVENTION

Ordinary removable dentures, dental plates and the like, comprise teeth mounted in a suitable plate or base. While dentures are traditionally fitted for the individual user, the fit can change over time which may result in slippage or discomfort. Denture adhesives are used to temporarily adhere the dentures to the surfaces of the oral cavity, in particular the oral mucosa. Denture adhesives are typically applied to either the denture or oral surface at the beginning of the day when the dentures are placed into the oral cavity, and the adhesives tend to bio-erode during the course of the day due to the action of saliva and chewing.

Considerable effort has been made over the years to develop improved denture adhesive products. Both synthetic and natural polymers and gums have been used alone and in combination with various adhesives and other materials in an attempt to improve hold and reduce oozing of the adhesive from under the dental plate, and to avoid messiness and difficulty of removing the residual adhesive from the mouth and dentures after use. For example, alkyl vinyl ether-maleic copolymers and salts thereof are known for providing hold in denture adhesive compositions. Such disclosures include: U.S. Patent 3,003,988, Germann et al., issued October 10, 1961; U.S. Patent 4,980,391, Kumar et al., issued December 25, 1990; U.S. Patent 5,073,604, Holeva et al., issued December 17, 1991; U.S. Patent 5,525,652, Clarke, issued June 11, 1996; U.S. Patent 5,340,918, Kittrell et al., issued Aug. 23, 1994; U.S. Patent 5,830,933, Synodis et al., issued Nov. 3, 1998, WO 02/30317 A, Block Drug Co., Gasman Robert C., Ortiz Johny, Wong Eddie, issued April 18, 2002; WO 01/41710 A, Procter & Gamble, issued June 14, 2001; WO 01/15657 A, Procter & Gamble, issued March 8, 2001; WO 2005/081935 A, Smithkline Beecham Corp., Smetana Alfred J., Wilensky Stuart, issued September 9, 2005; US 2003/0108489 A1, Procter & Gamble, issued June 12, 2003.

In addition to adhesion, it is desirable to reduce oozing or to reduce the negative aesthetics of oozing experienced by the consumer. Oozing may occur due to seeping of the denture adhesive from under the dental plate in the oral cavity caused by a variety of factors including a low viscosity denture adhesive, use of too much denture adhesive, improper application of the denture adhesive on the denture plate, etc. When oozing occurs in the oral cavity, the denture adhesive composition is exposed to the oral cavity. Therefore, any negative taste, negative mouth-feel, or any other any other negative aesthetic associated with the denture adhesive composition may be more noticeable and objectionable to the consumer. Sources of such negative perception may include the denture adhesive polymer itself or salts of the denture adhesive polymer, including those crosslinked with zinc salts. Taste considerations are significant since denture adhesive compositions are used in the oral cavity for up to 6-7 hours or longer. Furthermore, consumers may stop using the adhesive or may tend to apply less adhesive the next time if they experience the negative perception of ooze. This may lead to decreased denture hold or decreased denture performance. This decrease in performance can mean less denture stability, denture retention, or an increase in food lodging itself under the denture prosthesis.

In accordance with the present invention, the denture adhesive composition herein will provide these improved denture adhesive properties including improved hold, fit, ease of handling, ease of application, decreased ooze, and/or improved clean up under the varied conditions of the oral cavity.

### SUMMARY OF THE INVENTION

The present invention relates to a denture adhesive article comprising:
a) a homogeneous, bioerodible mixture of from 10% to 90% by weight of the article of a denture adhesive component; and from 2% to 90% by weight of the article of a water insoluble thermoplastic component selected from polyethylene, microcrystalline wax, elastomers and mixture thereof; and
b) at least one non-adhesive self supporting substrate.

### BRIEF DESCRIPTION OF THE DRAWINGS

While the specification concludes with claims particularly pointing out and distinctly claiming the invention, it is believed that the present invention will be better understood from the following description taken in conjunction with the accompanying drawings in which:
Fig. 1 is a top plain view of an embodiment of a concave shaped denture adhesive article having symmetrical dimensions;
Fig. 2 is a top plain view of an embodiment of a concave shaped denture adhesive article having asymmetrical dimensions;

### DETAILED DESCRIPTION OF THE INVENTION

A detailed description of essential and optional components of the present invention is given below.

### DEFINITIONS

The abbreviation "cm", as used herein, means centimeter. The abbreviation "mm" as used herein, means millimeter. The abbreviation "g" as used herein, means gram.

The term "denture" and/or "denture prosthesis" as used herein refers to either the upper or lower denture, or both.

The term "denture adhesive article" and/or "article" as used herein refers to articles designed to fit, conform and adhere to contoured surfaces, such as a denture, as well as the gums or the roof of the mouth. The articles herein are substantially solid prior to use and can be picked up manually in substantially one piece and positioned on the denture.

The term "flexible" or "flexible article" as used herein means that a 0.67 mm thick piece of the article may be wrapped 180 degrees around a solid cylinder of 1 cm diameter without cracking upon visual observation.

The term "safe and effective adhesive amounts" as used herein means an amount sufficient to provide adherence to the oral cavity and/or provide adherence of a denture to the oral cavity, without toxicity to the user or damage to oral tissue.

By "safe and effective amount", as used herein, is meant an amount of an agent high enough to significantly (positively) modify the condition to be treated or positively modify the benefit sought, but low enough to avoid serious side effects (at a reasonable benefit/risk ratio), within the scope of sound medical/dental judgment. The safe and effective amount of an agent may vary with the particular condition being treated, the age and physical condition of the patient being treated, the severity of the condition, the duration of treatment, the nature of concurrent therapy, the specific form of the source employed, and the particular vehicle from which the agent is applied.

The term "AVE/MA" as used herein refers to alkyl vinyl ether-maleic acid or anhydride copolymer. The term "mixed polymer salts" or "mixed salts", as used herein, refers to salts of AVE/MA where at least 2 different cations are mixed on the same polymer with each other or with other salts.

The term "free acid" or "FA" component, as used herein, refers either to the unreacted carboxyl groups (-COOH) of AVE/MA copolymer plus any other monovalent cations of carboxyl groups (e.g., COONa) of the polymer. Monovalent cations include Group IA cations, such as sodium, potassium, hydrogen, etc. In one embodiment, the term "free acid" refers to the unreacted carboxyl groups (-COOH) of AVE/MA plus sodium and potassium cations. In another embodiment, the term "free acid" refers only to the unreacted carboxyl groups (-COOH) of the AVE/MA.

The term "toxicologically-acceptable", as used herein, is used to describe materials that are suitable in their toxicity profile for administration to humans and/or animals.

By "non-aqueous", as used herein, is meant that the article does not contain added water but may contain water that is included in another component as supplied commercially by the manufacturer.

The term "water-insoluble" as used herein refers to a material that, when exposed to an excess of water, does not dissolve, but may disperse to varying degrees. In some embodiments the term "water-insoluble" refers to a material that is less than about 10%, 5%, 2%, or 1% soluble in water.

The term "thermoplastic" as used herein refers to a material that melts, softens, and becomes more flexible, extrudable, deformable, shapable, moldable, flowable, processable, and/or changes rheology when exposed to heat. In one embodiment the material generally solidifies, hardens, and/or substantially returns to its original condition, when subsequently cooled.

The term "bioerodible" as used herein means that the article, when exposed to excess of water or saliva, will erode over time due to physical and/or chemical action. The time necessary to erode the article can be any length of time from instantaneous to five days, in one embodiment the time to erode is from about 1 to about 3 days. The article may erode completely or substantially, however ultimately the article will lose its original form and/or integrity. For example, after application and use for at least about 24 hours in the oral cavity the article will not have sufficient product integrity to easily separate or peel, in its original form, from the denture or oral surface. In one embodiment the article bioerodes such that no portion of the article remains on the denture or mouth after the article has been used in the oral cavity for about 24 hours. In another embodiment some portion or residue from the article remains on the denture or oral surface after removing the denture from the oral cavity; however, this portion or residue from the article can be cleaned by brushing away with a toothbrush, but not easily separated from the denture.

The percentages used herein to describe the cationic salt function of the alkyl vinyl ether-maleic acid or anhydride copolymers are defined as the stoichiometric percent of the total initial carboxyl groups reacted on the polymer.

All other percentages used herein are by weight of the article unless otherwise indicated.

All measurements referred to herein are made at 25°C unless otherwise specified.

All percentages, ratios, and levels of ingredients referred to herein are based on the actual amount of the ingredient, and do not include solvents, fillers, or other materials with which the ingredient may be combined as a commercially available product, unless otherwise indicated.

All publications, patent applications, and issued patents mentioned herein are hereby incorporated in their entirety by reference. Citation of any reference is not an admission regarding any determination as to its availability as prior art to the claimed invention.

To the extent that any meaning or definition of a term in this written document conflicts with any meaning or definition of the term in a document incorporated by reference, the meaning or definition assigned to the term in this written document shall govern.

### DENTURE ADHESIVE ARTICLE

The present invention relates to denture adhesive articles (e.g. shaped) designed to fit, conform and adhere to contoured surfaces such as a denture as well as the gums or the roof of the mouth.

In one embodiment the articles herein minimize or avoid the problem of premature sticking during application of the article to the denture. That is, with some prior art denture adhesive articles, before the article can be properly positioned over a target surface on the denture, inadvertent contact of the article with the denture may cause premature sticking at one or more locations on the denture. This may inhibit proper positioning of the article. Premature sticking may also cause contamination or degradation of the article prior to final positioning on the denture.

In one embodiment the term "dry tack" as used herein means that present articles exhibit minimal and/or no adhesive or cling properties in the dry state until activated by pressure applied by a user. In one embodiment this characteristic permits the present articles to be stored and dispensed in any desired mode without encountering the difficulties of premature clinging or adhering to themselves, and without the need for separate release sheets, liners, spacers, or the like. At the same time, in one embodiment when pressure activated at the desired location and at the desired time, the articles can, in the dry state, exhibit sufficient adhesive properties to form a bond to most plastic surfaces including a denture surface, this bond being sufficiently strong to survive handling of the denture without bond failure. Therefore, in one embodiment the articles herein, in the dry state, adhere to a target denture surface only when pressed thereagainst, thereby minimizing or avoiding this problem of inadvertent adherence during positioning on the denture surface. In one embodiment then, the articles herein do not have to be moistened or wet prior to application to the denture, thus providing a simple and easy way to apply an article to the denture.

In one embodiment the term "dry tack" as used herein means that present articles exhibit minimal and/or no adhesive or cling properties until activated by pressure applied by the user after the article has been warmed by the hands of the user, potentially during the course of application of the article to the denture surface.

In another embodiment the articles herein are non-tacky to the touch prior to application to the denture.

In another embodiment the term "dry tack" as used herein means articles herein in a dry and un-wetted state, are capable of immediate bonding by surface attachment to a dry plastic, polymethyl methacrylate, and/or other denture prothesis substrate, upon subjecting the article to pressure. In one embodiment the dry article, develops bonding by surface attachment to a dry denture prosthesis substrate upon the application of finger pressure whereby the article remains bonded under its own weight, and the article herein will not remain bonded to this dry substrate under its own weight without using finger pressure to apply the article to the substrate. In one embodiment the force or pressure may be generated by one or more fingers. This force or finger pressure, in one embodiment, may be applied for 1-10 seconds or longer. In another embodiment the bonding of the article to the substrate is maintained from about 10 seconds to about 3 minutes or longer, in another embodiment from about 30 seconds to about 1 minute or longer.

In one embodiment the dry tack of the article is from about 0.025, 0.1, 1, 10, 100, 1000 gram force/square centimeter to about 10, 100, 1000, 10,000, 50,000, 100,000 gram force/square centimeter and any combination thereof.

In one embodiment the dry tack of an article that can be repositioned is from about 0.025 grams/force square centimeter to about 0.30 grams/force square centimeter, and in another embodiment from about 0.025 gram force/square centimeter to about 0.25 gram force/square centimeter.

It is reported that the room temperature modulus of any tacky adhesive is *less* than 3 x 10⁶ dynes/cm² when measured at a frequency of 1 Hz. This finding is a criterion for tack and has been given the name "Dahlquist criterion for tack."(Adhesion and Adhesives Technology, by Alphonsus Pocius, 2^{nd} Edition, 2002 Carl Hanser Verlag, Munich).

In one embodiment of the current invention, the article has a modulus greater than the 'Dahlquist criterion for tack' of about 3 x 10⁶ dynes/cm². In another embodiment, the article has a shear storage modulus G' (measured in dynes/cm² at a frequency of about 1Hz at about 25C) greater than about 5 x 10⁶; in another embodiment greater than about 1 x 10⁷; in another embodiment greater than about 5 x 10⁷ ; and in another embodiment greater than about 8 x 10⁷.

In one embodiment the article has a shear storage modulus G' (measured in dynes/cm² at a frequency of about 1Hz at about 25C) from about 1 x 10⁶, 3 x 10⁶, 5 x 10⁶, 1 x 10⁷, 5 x 10⁷, and 8 x 10⁷ to about 5x 10⁸, 5 x 10⁷, 1 x 10⁸, 5 x 10⁹, 1 x 10⁹, and 1 x 10¹⁰ and/or any combination thereof.

In one embodiment the article has a flexural stiffness of less than about 10 grams/cm, in another embodiment less that about 5 grams/cm, in another embodiment less that about 3 grams/cm, in another embodiment less than about 2 grams/cm and in yet another embodiment from about 0.1, 0.5, 1, to about 2, 3, 5, 10 grams/cm, in any combination, flexural stiffness as measured on a Handle-O-Meter, model #211-300, available from Thwing-Albert Instrument Company of Philadelphia, PA as per test method ASTM D2923-95.

In one embodiment the articles herein have a normalized dislodgement force of from about 1100 to about 12,000 grams per sq.cm, in another embodiment from about 1300 to about 10,000 grams per sq.cm, in another embodiment from about 1200 to about 5000 grams per sq.cm, in another embodiment from about 1400 to about 5000 grams per sq.cm, in another embodiment from about 1300 to about 2500 grams per sq.cm, in another embodiment from about 1750 to about 2500 grams per sq.com. In another embodiment the normalized dislodgement force is from about 1100, about 1200, about 1300, about 1400, about 1500, about 1750 grams per sq.cm. to about 12,000, about 10,000, about 7500, about 5000, about 2500, about 2250 grams per sq.cm, and/or any combination thereof. In one embodiment the dislodgement force ratio is from about 1.1, 1.2, 1.3, 1.4, 1.5, 1.6, 1.7, 1.8, 1.9, 2.0 to about 1.3, 1.4, 1.5, 1.6, 1.7, 1.8, 1.9, 2.0, 3, 4, 5, 6, 8, 10, and/or any combination thereof. In one embodiment the dislodgement force ratio is from about 1.1 to about 10, from about 1.1 to about 8, from about 1.3 to about 4, and/or from about 1.3 to about 2.5.

In one embodiment the articles herein are substantially solid prior to use and can be picked up manually, in substantially one piece, and positioned on the denture. Additionally, in one embodiment the articles are capable of being picked up manually, and positioned on the denture, resulting in little or no residue on the fingers. In another embodiment the articles comprise a single layer. In yet another embodiment the articles are laminates, and/or composites.. In one embodiment the articles are pre-shaped and/or preformed. In another embodiment the articles herein may be dispensed by the consumer via a unit dose package, multi-dose package, and/or a sachet.

In another embodiment the denture adhesive article comprises a solvent. The solvent may be subsequently dissipated, by evaporation, bio-absorption, dispersion, dissolution, etc. In another embodiment the above mentioned optional solvent is also miscible with the water insoluble component. In one embodiment, the denture adhesive article only minimally oozes out from under the denture. In one embodiment the denture adhesive article has a "normalized ooze amount" from about 0%, 0.00001%, 0.001%, 5%, 10%, 15%, 20%, 25%, 30% to about 15%, 20%, 25%, 30%, 40%, 50% and/or any combination thereof. In one embodiment the denture adhesive article has a "ooze ratio" from about 0, 0.00001, 0.001, 0.01, 0.1, 0.2, 0.3, 0.4, 0.5, 0.6 to about 0.3, 0.4, 0.5, 0.6, 0.7, and/or any combination thereof

Regardless of the form of dispensing the article, including but not limited to pre-dosed ready to use articles the articles are substantially solid prior to use and can be picked up manually.

Some denture adhesive articles are pre-dosed and/or ready to use. A user may be able to identify these items visually as a denture adhesive article, as they are often in the form of a strip contained within a package. However, if not evident that these denture adhesive products are articles, these denture adhesive articles can be identified as articles by the following method:
1. Shape composition into a sheet about 0.67 mm thick x about 8 mm wide x about 44 mm long.
2. Place sheet on a denture-tile.
3. Use fingers to pick up sheet.
4. The composition is an article if it can be picked up in substantially one piece.

Substantially in one piece means, as used herein, that from about 75, 80, 85, 90% to about 100, 90, 85, 80, 75, 70% and/or any combination thereof of the denture adhesive composition remains in one piece when manually picked up from the denture surface.

In addition to the aforementioned test methods denture adhesive articles can also be identified as articles by the amount of ooze, as determined by the ooze method (as defined herein). In addition to being able to be manually picked up and moved in substantially one piece, a denture adhesive article has a normalized ooze amount of from about 0, 3, 5, 10, 15, 20, 25% of the total composition to about 30, 25, 20, 15, 10, 5, 3 % of the total composition and/or any combination thereof and/or the ooze ratio is from about 0, 00001, 0.001, 0.01, 0.1, 0.2, 0.25, 0.3, to about 0.1, 0.2, 0.25, 0.3, 0.4, 0.5 and/or any combination thereof.

In one embodiment the article herein comprises ingredients of natural origin.

In one embodiment the article herein comprises a homogeneous mixture of the denture adhesive component and the water insoluble thermo-plastic component.

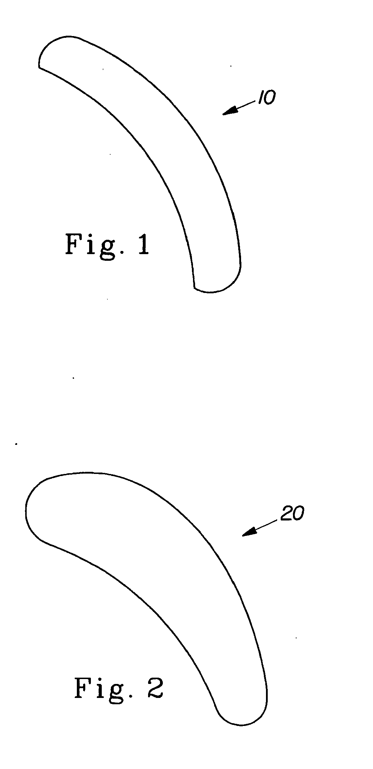

The denture adhesive article can have a variety of shapes and sizes including but not limited to a concave shape which is either symmetrical or asymmetrical. Fig. 1 shows a symmetrical concave shaped denture adhesive article 10. Fig. 2 shows an asymmetrical conclave shaped denture adhesive article 20, in particular Fig. 2 shows a kidney shaped denture adhesive article 20.

### DENTURE ADHESIVE COMPONENT

The present invention comprises a safe and effective adhesive amount of a denture adhesive component, generally at a level of from about 10% to about 90%, in another embodiment from about 15% to about 70%, in another embodiment from about 20% to about 70%, in yet and in another embodiment from about 25% to about 65%, and in yet another embodiment from about 30% to about 65%, by weight of the article. In one embodiment the articles of the present invention comprise from at least 20 percent by weight, and in another embodiment at least 30 percent by weight of the article, of a denture adhesive component.

In one embodiment the denture adhesive components herein are mucoadhesive, hydrophilic, water soluble, have the property of swelling upon exposure to moisture, and/or to form a mucilaginous mass when combined with moisture. In one embodiment the denture adhesive components are selected from the group consisting of natural gums, synthetic polymeric gums, AVE/MA, salts of AVE/MA, AVE/MA/IB, salts of AVE/MA/IB, copolymer of maleic acid or anhydride and ethylene and salts thereof, copolymer of maleic acid or anhydride and styrene and salts thereof, copolymer of maleic acid or anhydride and isobutylene and salts thereof, polyacrylic acid and polyacrylates thereof, polyitaconic acid and salts thereof, mucoadhesive polymers, water-soluble hydrophilic colloids, saccharide derivatives, cellulose derivatives, and mixtures thereof. Examples of such materials include karaya gum, guar gum, gelatin, algin, sodium alginate, tragacanth, chitosan, acrylamide polymers, carbopol, polyvinyl alcohol, polyamines, polyquartemary compounds, polyvinylpyrrolidone, cationic polyacrylamide polymers, AVE/MA, AVE/MA/IB, mixed salts of AVE/MA, mixed salts of AVE/MA/IB, polymeric acids, polymeric salts, polyhydroxy compounds, and mixtures thereof.

In one embodiment the denture adhesive components are selected from the group consisting of salts of AVE/MA, mixed salts of AVE/MA, cellulose derivatives (such as methylcellulose, carboxymethylcellulose, hydroxyethylcellulose, hydroxypropylcellulose, hydroxy-propylmethylcellulose, and mixtures thereof), polyethylene glycol, karaya gum, sodium alginate, chitosan, corn starch, and mixtures thereof. In yet another embodiment, the adhesive component is selected from the group consisting of mixed salts of AVE/MA, cellulose derivatives, and mixtures thereof.

In one embodiment the denture adhesive component is not thermoplastic and/or comprises only low levels of water soluble thermoplastic polymers, from about 0.01 to about 5% of water soluble thermoplastic polymer such as polyethylene oxide, hydroxypropyl cellulose, hydroxyproplymethylcellulose; polyethylene glycol; in another embodiment from about 0.01 to about 1% of water soluble thermoplastic polymer, or is essentially free of water soluble thermoplastic polymers.

### Alkyl Vinyl Ether-Maleic Copolymer

In one embodiment of the invention the denture adhesive component is AVE/MA or salts of AVE/MA. The alkyl vinyl ether-maleic acid co-polymer comprises or consists essentially of the repeated structural unit: 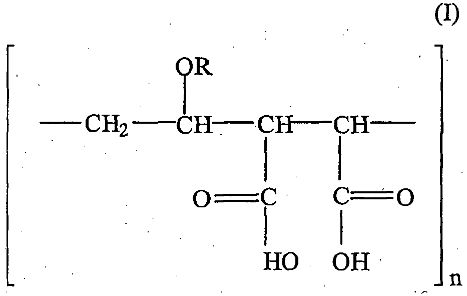 wherein R represents an alkyl radical, in one embodiment a C₁ to C₅ alkyl radical, n is an integer greater than one representing the number of repeated occurrences of the structural unit in a molecule of the polymer.

In one embodiment, the adhesive component is AVE/MA and salts thereof, preferably mixed salts of AVE/MA, wherein the copolymer contains a cationic salt function comprising a cation selected from the group consisting of Group IA and Group 2A cations of the periodic table, yttrium, titanium, zirconium, vanadium, chromium, manganese, iron, nickel, copper, zinc, boron, aluminum, and mixtures thereof. In another embodiment, the adhesive component is a mixed salt of AVE/MA containing a cationic salt function comprising a cation selected from the group consisting of strontium, zinc, iron, boron, aluminum, vanadium, chromium, manganese, nickel, copper, yttrium, titanium, magnesium, calcium, sodium, and mixtures thereof, and in yet another embodiment the cation is selected from the group consisting of strontium, zinc, iron, magnesium, calcium, sodium, and mixtures thereof.

AVE/MA contains, in one embodiment, a cationic salt function comprising from about 5% to about 50%, in another embodiment, from about 10% to about 40%, in yet another embodiment, from about 10% to about 35% (of the total initial carboxyl groups reacted) zinc cations. These zinc cations can be mixed with other cations selected from the group consisting of: from about 5% to about 65%, preferably from about 10% to about 60%, strontium cations, from about 0.001% to about 2.5%, preferably from about 0.01% to about 2% of iron, boron, aluminum, vanadium, chromium, manganese, nickel, copper, yttrium, and/or titanium cations, from about 5% to about 65%, preferably from about 15% to about 50% of calcium and/or magnesium cations and/or sodium cations.

AVE/MA and salts thereof, are also, described in U.S. Patent Nos. 5,073,604 to Holeva et al., issued 12/17/91; 5,525,652, issued Jun. 11, 1996, Clarke et al.; 6,025,411, issued Feb. 15, 2000, Wong et. al.; 4,758,630, issued July 19, 1988, Shah et al.; 5,304, 616, issued April 19, 1994, Rajaiah et al.; 5,424,058, issued June 13, 1995, Rajaiah; 5,424,058, issued 6/13/95, Rajaiah et al.; 4,758,630, issued July 19, 1988, Shah et al.; 5,830,933, issued Nov. 3, 1998, Synodis et al.; 2,047,398, issued July 14, 1936, Voss et al.; 3,003,988, issued Oct. 10, 1961, Germann et al.; 5,880,172, Rajaiah et al., issued March 9, 1999; 5,900,470, Prosise et al., issued 5/4/99; 5,037,924, Tazi et al., issued 8/6/91; 5,082,913, Tazi et al, issued 1/21/92; all of which are incorporated herein by reference in their entirety. Salts of AVE/MA are also described in P&G copending applications U.S. Patent Nos. 6,355,706 to Rajaiah, et al., issued March 12, 2002; 6,617,374 to Rajaiah, et al., issued September 9, 2003.

In one embodiment the free acid level of the salts of the AVE/MA or AVE/MA/IB is at least about 36%, in another embodiment is from about 36% to about 60%, and even in another embodiment is from about 40% to about 55%, of the total initial carboxyl groups of the copolymer.

In one embodiment the specific viscosity of the starting copolymer acid or copolymer anhydride is from about 1.2 to about 14, when preferably measured in a 1% weight/volume solution in MEK (methyl ethyl ketone) at 25°C. Other methods and solvents can be used to measure the specific viscosity such as a 1% weight/volume solution in DMF (dimethyl formamide) at 25°C and a 1% weight/volume solution in 2-butanone at 25°C.

Suitable AVE/MA copolymers may be prepared by well-known methods of the prior art; see, for example, US 2,782,182, and US 2,047,398.

Methods of making mixed salts of AVE/MA polymers are further disclosed in U.S. Patent Nos. 5,073,604, Holeva et al., issued Dec. 17, 1991 and 5,872,161, Liang et al., issued Feb. 16, 1999.

### WATER INSOLUBLE COMPONENT

The present article comprises a safe and effective amount of a water insoluble component. This component is at a level from about 2, 5, 10, 20, 25, 30, 35% to about 45, 50, 60, 70, 90%, and/or any combination thereof to create ranges, by weight of the article, in particular the water insoluble component level is from 2% to 90% from about 20% to about 70%, from about 25% to about 60%, or from about 35% to about 60% by weight of the article. In yet another embodiment the water insoluble component is both water insoluble and substantially non-swellable in water.

In one embodiment the water insoluble component is a water insoluble thermoplastic component that is selected from the group consisting of elastomers, polyethylene, microcrystalline wax, and mixtures thereof; in another embodiment is selected from the group consisting of polyethylene, microcrystalline wax, and mixtures thereof.

In one embodiment the water insoluble thermoplastic component comprises elastomers such as Ethylene-Ethylene-Propylene rubber, Ethylene-Propylene rubber, Styrene-Ethylene-Ethylene-Ethylene-Propylene-Styrene rubber, and combinations thereof; and these may optionally be further combined with waxes.

In one embodiment the article herein has a water insoluble thermoplastic component having a penetration of about 25 to about 350; in another embodiment from about 27 to about 250 in another embodiment from about 30 to about 150, and in another embodiment from about 40 to about 150, and in another embodiment from about 50 to about 80, and in yet another embodiment from about 60 to about 80. The penetration values are from about 25 to about 250 when measured using the ASTM D1321 method and the penetration values are from about 25 to about 350 when measured using the ASTM D937 method, both are existing methods known in the art. Although ASTM D937 and ASTM D1321 generally are used to measure the penetration of petrolatum or petroleum waxes, respectively, these methods may be used to measure the penetration of waxes derived from petroleum and other types of water insoluble thermoplastic components with appropriate modifications, for example, these other components may need to be melted at higher temperature which will be apparent to one of skill in the art.

In obtaining the above penetration values, water insoluble thermoplastic component that are outside of these penetration values may be mixed with another ingredient that modifies the penetration of the thermoplastic component. Therefore the water insoluble thermoplastic component may be a single ingredient or may be a mixture of ingredients. For example, Multiwax W 180 manufactured by Witco (Crompton, Sonneborn), having a penetration value of about 15 to about 20 may be mixed with petrolatum (at a 1:1 ratio) to raise the penetration value to above 25.

In an embodiment the wax is microcrystalline wax manufactured by Crompton, Sonneborn (Witco) and referred to and sold under the trademark MutiwaxW-835. This wax has a melt point ranging from about 73.9 ° C to about 79.4 °C (ASTM D127), has a penetration at 25° of from about 60 to about 80 (ASTM D1321), has a kinematic viscosity at 98.9 °C, of from about 75 to about 90 (ASTM D2161), has a flash point, COC, of about 246 °C Min. (ASTM D92), and has a congealing point, of about 77 °C (ASTM D938).

In another embodiment the water insoluble thermoplastic component is polyethylene such as A-C 1702 and A-C 6702 made by Honeywell, with a penetration value of 98.5 and 90.0, respectively, under ASTM D1321.

In one embodiment if the article contains polyethylene oxide, then either the water insoluble component is thermoplastic or the article may not include a fibrous paper web or paper laminate.

In one embodiment the article herein is substantially free of honey mixed with alcohol. In another embodiment the article is substantially free of polyvinyl acetate resin in ethyl alcohol.

### Non-Adhesive Substrate

In one embodiment the present invention comprises at least one non-adhesive self supporting substrate. In one embodiment the non-adhesive self-supporting substrate is characterized by its ability to maintain strength and provide integrity for the article in the presence of water and/or saliva.

In one embodiment, the user or consumer is able to easily peel away the article in its original form, from the prosthesis after the article is used in the oral cavity as a denture adhesive.

In one embodiment the non-adhesive self-supporting substrate is a solid substrate material having a penetration value of less that about 20, in another embodiment less than about 10, and in another embodiment less than about 5, based on ASTM D1321 or ASTM D937. In one embodiment the non-adhesive self-supporting substrate is a solid substrate material having a penetration value of about 0.5, 1, 2 to about 10, 15, 18, 20, or any combination of these, based on ASTM D1321 or ASTM D937.

In one embodiment the non-adhesive self-supporting substrate is a substrate with a hardness value of greater than about 0.5, in another embodiment greater than about 1, and in another embodiment greater than about 2, under ASTM D5. In one embodiment the non-adhesive self-supporting substrate is a solid substrate material having a hardness value of about 0.5, 1, 2 to about 10, 15, 18, 20, or any combination of these, based on ASTM D5.

In one embodiment the non-adhesive self-supporting substrate may include materials such as polyester, polypropylene, nylon, rayon, cellulose acetate, non-adhesive cellulose derivatives, cellulose acetate, cloth, fibrous fleece, paper, plastic, leather, microcrystalline wax, synthetic fibers, natural fibers, and mixtures thereof. In one embodiment the non-adhesive substrate is selected from the group consisting of non-adhesive cellulose derivatives, polyester, polypropylene, nylon, rayon, cloth, paper, microcrystalline wax, and mixtures thereof. In another embodiment the non-adhesive substrate is selected from the group consisting of polyester, polypropylene, rayon, nylon, cloth, paper, and mixtures thereof; in another embodiment is polyester.

In one embodiment the non-adhesive self-supporting substrate may be in any physical form suitable for providing strength and/or integrity to the present denture adhesive component and/or water insoluble thermoplastic component. Such physical forms include extruded films and/or sheets, non-woven, woven, continuous, chopped, foam, and combinations thereof. In addition, the non-adhesive self-supporting layer may be formed by any process commonly known in the art. Such processes include extrusion, casting, calendaring, coating, un-bonded, spraybonded, spun-bonded, needle-punched, carded, thermal bonded hydroentangled, meltblown, aperture print bonded, needled, wet-laid, dry-laid, and combinations thereof.

In one embodiment the non-adhesive self supporting substrate has a flexural stiffness of less than about 10 grams/cm, in another embodiment less that about 5 grams/cm, in another embodiment less that about 3 grams/cm, in another embodiment less than about 2 grams/cm and in yet another embodiment from about 0.1, 0.5, 1, to about 2, 3, 5, 10 grams/cm, in any combination, flexural stiffness as measured on a Handle-O-Meter, model #211-300, available from Thwing-Albert Instrument Company of Philadelphia, PA as per test method ASTM D2923-95. Flexural stiffness is a material property that is a function of a combination of film/strip thickness, width and material modulus of elasticity. This test is a method for measuring the rigidity of polyolefin film and sheeting. It determines the resistance to flexure of a sample by using a strain gauge affixed to the end of a horizontal beam. The opposite end of the beam presses across a strip of the sample to force a portion of the strip into a vertical groove in a horizontal platform upon which the sample rests. A microammeter wired to the strain gauge is calibrated in terms of deflection force. The rigidity of the sample is read directly from the microammeter and expressed as grams per centimeter of the sample strip width.

### MISCELLANEOUS OPTIONAL INGREDIENTS

### Plasticizing Agent

The articles of the present invention may also optionally comprise a safe and effective amount of one or more toxicologically-acceptable plasticizers. In one embodiment the level of the plasticizing agent ranges from about 0.0% to about 40%, in one embodiment from about 0.01 % to about 40%, in another embodiment from about 1% to about 10%, in another embodiment from about 2% to about 5%, by weight of the article. In yet another embodiment the denture adhesive article does not comprise a plasticizer.

Suitable plasticizing agents of the present invention include, but are not limited to, polyols (such as sorbitol); glycerin; propylene glycol; acetylated monoglyceride; hydrogenated starch hydrolysates; corn syrups; and derivatives thereof; xylitol, glycerol monoesters with fatty acids; triacetin; diacetin; and monoacetin; dimethyl phthalate; diethyl phthalate; dioctyl phthalate; diethylene glycol; triethylene glycol; tricresyl phosphate; dimethyl sebacate; ethyl glycolate; ethylphthalyl ethyl glycolate; o- and p-toluene ethyl sulfonamide; phthalic acid derivative, glycerol triacetate, citric acid derivative, phosphoric acid derivative, glycol, glycol derivative, paraffin wax, a pentaerythritol ester of a fatty acid, stearic acid derivative, glycerol monostearate, polyethylene glycol, butyl phthalyl butyl glycolate, butyl phthalyl butyl glycolate, dimethyl phthalate, dibutyl phthalate, triacetin, triethyl citrate, acetyl triethyl citrate, acetyl tributyl citrate, triphenyl phosphate, diethylene glycol, caprylic triglyceride, capric triglyceride, propylene glycol dicaprylate/caprate and/or combinations thereof.

In another embodiment the plasticizer is water insoluble.

In one embodiment, the denture adhesive article, when extruded thermoplastically, does not cure and set as a result of the action of the plasticizer component. In another embodiment the plasticizer component does not solidify the water insoluble component or the denture adhesive article. In another embodiment the water insoluble thermoplastic component does not cure and set.

Alternatively, in one embodiment the denture adhesive article may be substantially free of plasticizers. In one embodiment the denture adhesive article can be substantially free of polyethylmethacrylate, triacetin, phthalic acid derivative, glycerol triacetate, citric acid derivative, phosphoric acid derivative, glycol, glycol derivative, paraffin wax, a pentaerythritol ester of a fatty acid, stearic acid derivative, glycerol monostearate, polyethylene glycol, butyl phthalyl butyl glycolate, butyl phthalyl butyl glycolate, dimethyl phthalate, dibutyl phthalate, triacetin, triethyl citrate, acetyl triethyl citrate, acetyl tributyl citrate, triphenyl phosphate, diethylene glycol, caprylic triglyceride, capric triglyceride, propylene glycol dicaprylate/caprate and/or combinations thereof.

### Gellant Agents

The articles of the present invention may also optionally comprise a safe and effective amount of one or more toxicologically-acceptable gellants. In one embodiment the level of the gellant agent ranges from about 0.01 % to about 40%, in another embodiment from about 1% to about 10%, in another embodiment from about 2% to about 5%, by weight of the article. Suitable gellant agents of the present invention include, but are not limited to, polyvinylpyrrolidone/eicosene copolymer sold under the tradename Ganex V-220F from ISP; tricontanyl polyvinylpyrrolidone sold under the tradename Ganex WP-660 from ISP; polyamide gallants including Sylvaclear, Sylvacote, Sylvagel, Unclear all available from Arizona Chemical including Sylvaclear Lightwax; Sylvaclear PA 20; Sylvaclear PA 30; Sylvaclear PA 50; Sylvacote 2228; Sylvacote 2228E; Sylvagel 5000; Sylvagel 6000; Uniclear 100; Uniclear 100VG; Uniclear 80; Uniclear 80V; and mixtures thereof.

### Flavors, Fragrance, Sensates

The articles of the present invention may also include one or more components which provide flavor, fragrance, and/or sensate benefit (warming or cooling agents). Suitable components include menthol, wintergreen oil, peppermint oil, spearmint oil, leaf alcohol, clove bud oil, anethole, methyl salicylate, eucalyptol, cassia, 1-8 menthyl acetate, sage, eugenol, parsley oil, oxanone, alpha-irisone, marjoram, lemon, orange, propenyl guaethol, cinnamon, vanillin, thymol, linalool, cinnamaldehyde glycerol acetal known as CGA, and mixtures thereof, as well as coolants. The coolant can be any of a wide variety of materials. Included among such materials are carboxamides, menthol, ketals, diols, and mixtures thereof. In one embodiment the coolants in the present articles are selected from the group consisting of the paramenthan carboxyamide agents such as N-ethyl-p-menthan-3-carboxamide, known commercially as "WS-3", N,2,3-trimethyl-2-isopropylbutanamide, known as "WS-23," and mixtures thereof. Additional preferred coolants are selected from the group consisting of menthol, 3-1-menthoxypropane-1,2-diol known as TK-10 manufactured by Takasago, menthone glycerol acetal known as MGA manufactured by Haarmann and Reimer, and menthyl lactate known as Frescolat® manufactured by Haarmann and Reimer. The terms menthol and menthyl as used herein include dextro- and levorotatory isomers of these compounds and racemic mixtures thereof. TK-10 is described in U.S. Pat. No. 4,459,425, Amano et al., issued 7/10/84. WS-3 and other agents are described in U.S. Pat. No. 4,136,163, Watson, et al., issued Jan. 23, 1979. These agents may be present at a level of from about 0% to about 40%, in another embodiment from about 0.05 to about 5%, and in another embodiment from about 0.1 to about 2%, by weight of the article.

### Other Optional Ingredients

The denture adhesive articles may also comprise one or more therapeutic actives suitable for topical administration. Therapeutic actives may be present at a level of from about 0% to about 70%, by weight of the article, and in one embodiment from about 1% to about 20% by weight of the article. Therapeutic actives include antimicrobial agents such as iodine, triclosan, peroxides, sulfonamides, bisbiguanides, or phenolics; antibiotics such as tetracycline, neomycin, kanamycin, metronidazole, cetylpyridium chloride, domiphen bromide, or clindamycin; anti-inflammatory agents such as aspirin, acetaminophen, naproxen and its salts, ibuprofen, ketorolac, flurbiprofen, indomethacin, eugenol, or hydrocortisone; dentinal desensitizing agents such as potassium nitrate, strontium chloride or sodium fluoride; fluorides such as sodium fluoride, stannous fluoride, MFP; anesthetic agents such as lidocaine or benzocaine; anti-fungals such as those for the treatment of *candida albicans;* aromatics such as camphor, eucalyptus oil, and aldehyde derivatives such as benzaldehyde; insulin; steroids; herbal and other plant derived remedies; and baking soda. It is recognized that in certain forms of therapy, combinations of these agents in the same delivery system may be useful in order to obtain an optimal effect. Thus, for example, an antimicrobial and an anti-inflammatory agent may be combined in a single delivery system to provide combined effectiveness.

Other suitable ingredients include colorants, preservatives (such as methyl and propyl parabens), thickeners such as silicon dioxide, and polyethylene glycol. Colorants, preservatives, thickeners may be present at levels of from about 0% to about 20%, by weight of the article, in another embodiment from about 0.1% to about 10%, by weight.

Additionally, the articles may also comprise one or more solvents. These optional solvents may be miscible with the water insoluble component and/or be capable of being dissipated in-situ. In one embodiment these solvents may be dissipated in-situ by evaporation, dissolution, dispersion, bio-absorption, or any other suitable means. In another embodiment these solvents may be dissipated in-situ to leave behind a denture adhesive article. Such solvents may include materials with a viscosity ranging from 0.01, 0.1, 1, 5 centipoise 20°C, to 5, 10, 100, 1000 centipoise at 20°C in any combination of these levels. In one embodiment these solvents may be silicones, hydrocarbons, iso-dodecane, iso-hexadecane, iso-eicosane, and/or polyisobutene. Suitable grades of solvents include the Permethyl series (sold by Prespers Inc., New Jersey) such as Permethyl 97A, 99A, 101A, 102A, and mixtures thereof.

### PROCESS FOR PREPARATION OF THE ARTICLE

The articles utilized in accordance with the invention are formed by processes conventional in the arts, e.g. the film making industries such as casting, coating, calendaring, extrusion. In one embodiment the separate components of the article are melted and then blended in a mixing tank until a homogeneous mixture is achieved. Thereafter, the melted mixture may be cast to an acceptable thickness, on an appropriate substrate. Examples of such substrates include Mylar, continuous moving stainless steel belt (which may eventually entering a dryer section if needed), release paper and the like. The articles are then cooled. The articles may then be dried if needed, e.g. in a forced-air oven. The temperature of the drying air and length of drying time depend on the nature of the solvent utilized as is recognized in the art. Generally, the drying temperatures include a temperature between about 25°C and 140°C, in another embodiment from about 60° and 90° C for a duration of about 20 minutes to about 60 minutes, in another embodiment from about 30 to about 40 minutes. The article may then be cut into desired shapes with desired dimensions and then stacked and/or subsequently packaged.

In one embodiment, after processing, the article is then die-cut into desired shapes. These shapes may facilitate application of the article to the dentures.

In one embodiment in particular the present article is processed as follows: 1. melt the wax component; 2. mix the AVE/MA salt(s) with any other adhesive component; 3. add the adhesive mixture to the wax melt; 4. stir to made a homogeneous mixture; 5. pour the homogeneous mixture into mold or onto a suitable surface; 6. cool the mixture until it solidifies; 7. remove from substrate or mold or cut into the desired shape.

Another conventional film-making process known in the art is extrusion. This method is possible with films wherein the film forming ingredient comprises a variety of extrudable materials. The mechanical particulars of the extrusion process, e.g. the particular equipment utilized, the extruding force, the shape and temperature of the orifice and/or dies are considered to be within the skill of the art and can be varied in a known manner to achieve the physical characteristics of the articles described herein.

In one embodiment the thickness of the articles herein is generally between about 0.1 mm to about 2.5 mm, in another embodiment is from about 0.4 mm to about 1.5 mm thick, in another embodiment is from about 0.5 mm to about 1mm thick. The article may be thicker or thinner depending on the degree of cushioning desired by the user or wearer.

In one embodiment the articles herein may optionally be multiphase or have visually distinct phases. In another embodiment the articles herein may optionally have a release liner.

### ARTICLE USE

The present articles are generally applied to the denture prosthesis and thereafter the denture is secured to the oral cavity. In one embodiment the dentures are dried prior to application of the article. In one embodiment it is not necessary to wet the article and/or the denture prosthesis prior to applying it to the denture prosthesis in order to make the article stick to the denture prosthesis. The article may be applied to any suitable location on the prosthesis. In one embodiment the denture wearer generally wears the article from about 1 hour to about 3 days, in another embodiment from about 6 hours to about 24 hours. After usage the prosthesis is removed from the oral cavity, and any remaining article may be cleaned from the prosthesis, for example by gentle scrubbing with water and a brush.

The following examples further describe and demonstrate embodiments within the scope of the present invention. The examples are given solely for the purpose of illustration and are not to be construed as limitations of the present invention. Many variations of these are possible without departing from the spirit and scope of the invention

### EXAMPLES

### EXAMPLE I

| | A | B | C | D | E | F | G | H | I |
|---|---|---|---|---|---|---|---|---|---|
| | Grams | Grams | Grams | Grams | Grams | Grams | Grams | Grams | Grams |
| Ca/Zn AVE/MA Salt | 33.00 | 33.00 | 33.00 | 53.00 | | 33.00 | 10.00 | 28 | 24.5 |
| CMC | 20.00 | 20.00 | 20.00 | | 53.00 | 25.00 | 10.00 | 15 | 28.5 |
| AVE/MA Acid S-97 | | | 1.00 | | | | | | |
| Microcrystalline Wax W-835 | 46.92 | 47.00 | 47.00 | 47.00 | 47.00 | 42.00 | 80.00 | 45.52 | 46.92 |
| Flavors | | | 0.50 | | | | | 0.4 | |
| Sacharrin | 00.08 | | 0.16 | | | | | 0.08 | 0.08 |
| Colorants | | | 0.10 | | | | | | |
| Non-adhesive sel supporting layer² | 1 | 2 | 3 | 4 | 5 | 6 | 7 | 1 | 1 |
| Silica | | | | | | | | 1 | |
| Corn Starch | | | | | | | | 10 | |

| | | | | | | | | | |
|---|---|---|---|---|---|---|---|---|---|
| ¹Multiwax W 835 manufactured by Witco (Crompton, Sonneborn). The Multiwax W 835 may also be substituted with either Polyethylene A-C 1702 or Polyethylene A-C 6702. ²1 is polyester; 2 is polyethylene; 3 is cellulose acetate; 4 is cloth; 5 is paper; 6 is nylon; 7 is rayon. | | | | | | | | | |

The Microcrystalline Wax W-835 (or Polyethylene A-C 1702 or Polyethylene A-C 6702) is melted, and the other ingredients (except for the non-adhesive self supporting layer) are blended with it. The mixture is then made into sheets by any suitable means such as extrusion or rolling into sheets of suitable thickness such as 0.1mm, 0.25mm, 0.50mm, 0.67mm, 0.73mm, or 1.0mm. A non-adhesive self supporting layer is then sandwiched between 2 sheets , and bonded by heat and/or pressure. The final sheet is then cut into shapes suitable for application to dentures.

In the above examples, all or part of the Ca/Zn AVE/MA salt may be substituted with Mg/Zn/Na AVE/MA Salts and/or Ca/Na AVE/MA salts; all or part of the CMC may be substituted with caraggeenan, and/or suitable cellulose derivatives; all or part of the Microcystalline Wax W-835 may be substituted with Polyethylene A-C 1702 (available from Honeywell), and/or Polyethylene A-C 6702 (available from Honeywell); and/or, the amount of each ingredient may also be increased or decreased by up to about 50%. The articles in the above examples may be used in conjunction with each other to form multilayer articles formed by pressure and/or heat.

### EXAMPLE II

| | A | B | C | D | E | F | G |
|---|---|---|---|---|---|---|---|
| | Grams | Grams | Grams | Grams | Grams | Grams | Grams |
| Ca/Zn AVE/MA Salt | | | | 33.00 | 33.00 | 30.00 | 10.00 |
| CMC | 10.00 | 20.00 | 30.00 | 20.00 | 20.00 | | |
| AVE/MA Acid S-97 | | | | 1.00 | | | |
| Microcrystalline Wax³ W-180 | 90.00 | 80.00 | 70.00 | 30.00 | 23.50 | 70.00 | 90.00 |
| Flavors | | | | 0.50 | | | |
| Sacharrin | | | | 0.16 | | | |
| Colorants | | | | 0.10 | | | |
| Petrolatum | | | | 17.00 | 23.50 | | |
| Non-adhesive self supporting layer⁴ | 1 | 2 | 3 | 4 | 5 | 6 | 7 |

| | | | | | | | |
|---|---|---|---|---|---|---|---|
| ³ Multiwax W 180 manufactured by Witco (Crompton, Sonneborn). , ⁴ 1 is polyester; 2 is polyethylene; 3 is cellulose acetate; 4 is cloth 5 is paper; 6 is nylon; 7 is rayon. | | | | | | | |

The Microcrystalline Wax W-180 is melted, and the other ingredients (except the non-adhesive self supporting layer) are blended with it. The mixture is then made into sheets by any suitable means such as extrusion or rolling into sheets of suitable thickness such as 0.1mm, 0.25mm, 0.50mm, 0.67mm, 0.73mm, or 1.0mm. A non-adhesive self supporting layer is then sandwiched between 2 sheets , and bonded by heat and/or pressure. The final sheet is then cut into shapes suitable for application to dentures.

In the above examples, all or part of the Ca/Zn AVE/MA salt may be substituted with Mg/Zn/Na, AVE/MA salts and/or Ca/Na AVE/MA salts; all or part of the CMC may be substituted with hydroxyethylcellulose, Caraggeenan, and/or Karaya Gum; all or part of the Microcystalline Wax W-180 may be substituted with Microcrystalline Wax W-445 (also available from Witco); all or part of the Petrolatum may be substituted with Mineral Oil, and/or Polybutene; and/or the amount of each ingredient may also be increased or decreased by up to about 50%. The articles in the above examples may be used in conjunction with each other to form multilayer articles formed by pressure and/or heat.

### EXAMPLE III

| | A | B |
|---|---|---|
| | Grams | Grams |
| Ca/Zn AVE/MA Salt | 33 | 33 |
| CMC | 20.00 | 20.00 |
| Silica | 0.57 | 0.29 |
| Mineral Oil | 11.98 | 5.99 |
| Microcrystalline Wax⁵ W-835 | 23.5 | 35.25 |
| Petrolatum | 10.96 | 5.48 |
| Non-adhesive self supporting layer⁶ | 1-7 | 1-7 |

The Microcrystalline Wax W-835 is melted, and the other ingredients are blended with it. The mixture is then made into sheets by any suitable means such as extrusion or rolling into sheets of suitable thickness such as 0.1mm, 0.25mm, 0.50mm, 0.67mm, 0.73mm, or 1.0mm. A non-adhesive self supporting layer is then sandwiched between 2 sheets , and bonded by heat and/or pressure. The final sheet is then cut into shapes suitable for application to dentures.

In the above example, all or part of the Ca/Zn AVE/MA salt may be substituted with Mg/Zn/Na AVE/MA salts and/or Ca/Na AVE/MA salts; all or part of the CMC may be substituted with HEC, Caraggeenan, and/or Karaya Gum; all or part of the Microcystalline Wax W-835 may be substituted with Polyethylene A-C 1702 (available from Honeywell), and/or Polyethylene A-C 6702 (available from Honeywell); all or part of the Petrolatum may be substituted with Mineral Oil, and/or Polybutene.

### Example IV

| | A | B | C |
|---|---|---|---|
| | % | % | % |
| Ca/Zn AVE/MA Salt | 33 | 33 | 33 |
| CMC | 20 | 20 | 20 |
| Silica | 1.03 | 0.97 | 0.86 |
| Mineral Oil | 21.56 | 20.36 | 17.96 |
| Petrolatum | 19.72 | 18.62 | 16.43 |
| Sacharin | 0.01 | 0.01 | 0.02 |
| Microcrystalline Wax³ W-835 | 4.69 | 7.04 | 11.73 |
| Non-adhesive self supporting layer⁷ | 1-7 | 1-7 | 1-7 |

| | | | |
|---|---|---|---|
| ⁵ Multiwax W 835 manufactured by Witco (Crompton, Sonneborn). The Multiwax W 835 may also be substituted with either Polyethylene A-C 1702 or Polyethylene A-C 6702. ⁶ 1 is polyester; 2 is polyethylene; 3 is cellulose acetate; 4 is cloth; 5 is paper; 6 is nylon; 7 is rayon. ⁷ 1 is polyester; 2 is polyethylene; 3 is cellulose acetate; 4 is cloth; 5 is paper; 6 is nylon; 7 is rayon. | | | |

The Microcrystalline Wax W-835 is melted at about 95C, and the other ingredients are blended with it at about 65C. The mixture is then made into sheets by any suitable means such as extrusion or rolling into sheets of suitable thickness such as 0.1mm, 0.25mm, 0.50mm, 0.67mm, 0.73mm, or 1.0mm. A non-adhesive self supporting layer is then sandwiched between 2 sheets, and bonded by heat and/or pressure. The final sheet is then cut into shapes suitable for application to dentures.

### TEST METHODS

The bioerosion of the inventive articles can be measured by the following method: run a water source on top of the sample specimen for about 30 minutes while the specimen sits atop a wire mesh. The water source is a laboratory faucet adjusted such that the temperature is 39 ± 1 °C and the flow rate is 16 ± 1 ml/sec. Use a funnel to focus the flow and help dampen the effect of small pressure and temperature fluctuations within the water lines. The wire mesh grid has square openings approximately 0.09 inches x 0.09 inches and is placed 2.5 inches below the tip of the funnel where it is clamped to a metal ring for support. Sample specimens weighing 0.025 g are placed on the mesh and images are taken at 0, 10 and 30 minutes to follow bio-erosion of the specimen. After 30 minutes the wire mesh containing the remainder of the specimen is removed and heated for 1 hour at 60 °C under vacuum to remove all remaining water. After the heating period, final weights are taken to calculate weight loss due to bio-erosion. An average of 3 specimens per sample are used to calculate bio-erosion time and weight loss. The article is bioerodible if it does not leave behind visible residue, film, or sheet after about 30 minutes under these testing conditions., and/or if it cannot be easily separated or peeled away manually in one or more large pieces after about 30 minutes under these testing conditions, and/or if it leaves behind less than about 2, less than about 4, less than about 6, and/or less than about 8% by weight of residue (of the original weight of the article) after about 30 minutes under these testing conditions. The above bio-erosion test may also be conducted at various time-points up to 8 hours.

The dry tack can be measured by the following method: 1. remove the article from the package material; 2. place the article on the palate-portion of a dry, acrylic upper-denture with the teeth facing downward; 3. apply pressure with fingers for about 3 to 10 seconds; 4. thereafter remove finger pressure; 5. then invert the denture with the teeth facing upward. In one embodiment the article demonstrates dry tack if: i. The article does not stick to fingers during steps 1-2, ii. leaves little or no residue on the fingers in steps 3-4, and iii. in step-5, the article does not fall off of the denture, once inverted, for at least about 10-30 seconds, or at least about 1 minute.

In another embodiment the article demonstrates dry tack if: i. The article does not stick to fingers during steps 1-4, and ii. in step-5, the article does not fall off of the denture, once inverted, for at least about 10-30 seconds, or at least about 1 minute.

In another embodiment the article demonstrates dry tack if in step-5, the article does not fall off of the denture, once inverted, for at least about 10-30 seconds, or at least about 1 minute.

The dry tack of the inventive articles can also be measured by the following procedure:
a. Compress a 5mm diameter disc (0.67mm thick) sample of the article between a 1" diameter cylindrical probe (made from polymethylmethacrylate) and a flat sheet of polymethylmethacrylate with a 2000 gram-force for 2 seconds,
b. Pull off the probe at 1mm/second and record peak force,
c. Repeat procedure with no sample sandwiched between the two surfaces, and
d. Calculate: Dry Tack in grams/square centimeter = (Peak Force with Sample - Peak Force without sample) / Cross sectional area of sample disc.

In one embodiment the above procedure is repeated with an applied force of 250 gram-force in step-a and the tack measured in steps b-d;

The article has dry tack if the tack measured with a 250 gram-force applied force is less than about 25, 50,100,200, or 500 grams/square-centimeter, and the tack measured with a 2000 gram-force applied force is greater than about 200, 500, 1000, 2000, 5000, 10000, or 25000 grams/square-centimeter, and any combination of these levels.

The modulus G' of the inventive article can be measured by the following procedure:
a. Load a sample disc of 8mm diameter and 0.67mm thickness onto a ARES rheometer using a parallel plate fixture with a compressive_force of 500 grams. If the sample is flowable a sufficient amount of material is used to fill the 1 mm gap on a 25 mm diameter parallel plate fixture.
b. Set strain to be 0.02%, c. Measure G' at a sweep of frequencies including 1 Hz.

The normalized dislodgement force and dislodgement force ratio of the inventive article can be measured by the following method:
Instrument: An Instron model 5544 is used. The load cell is calibrated according to manufacturer's specifications annually. The choice of load cell is determined by having the forces generated by the adhesive fall within the recommended operating range for the load cell. This is typically between 10% - 90% of full capacity.
Test Fixtures: The geometry of a cylindrical probe and a flat plate are used as the test fixtures. The probe is made from PMMA, 0.2 sq.cm to 10 sq.cm in surface area. For the base plate, the same PMMA material is used but in sheet form, ¼" thick. This is cut into 6" x 6" plates to be clamped onto the Instron.
Hydrating Liquid: Artificial saliva containing low levels of various salts is used to hydrate the adhesive.

### Artificial Saliva Composition

| Ingredient | | Amount per Liter |
|---|---|---|
| K₂HPO₄ | | 4.2 g |
| KH2PO4 | | 3.2 g |
| KOH | | 2 pellets |
| Mineral Stock Solution | | 5 ml |
| -KCl | 8 g per 100 ml of Stock Solution | |
| -NaCl | 8 g | |
| -Na2SO4 | 0.264 g | |
| -MgCl2.6H2O | 0.7687 | |
| (or 0.36 g Anhydrous MgC12) | | |

Adhesive: 0.1 to 1.0 gram of adhesive is applied to the probe.

Hydration: The hydrating liquid (0.2 mL of artificial saliva to 2.0 ml) is pipetted onto the surface of the adhesive. The assembly is then permitted to hydrate for 20 minutes or more.

Test Method: Once the sample is hydrated, it is mounted onto the Instron and the test is carried out via computer control. The method is comprised of the following steps:
(a) Compression to 750 to 7500 g of force
(b) Hold at compression for 2 minutes
(c) Reduce compressive force to 200 g_{f}
(d) Hold (1 minute)
(e) Pull off at 1 mm/s
(f) Record Peak Dislodgement Force
(g) Calculate "Normalized Dislodgement Force" = (Peak Dislodgement Force) / (Surface Area of Probe); report in grams force per sq.cm
(h) Repeat steps A-F for commercial Fixodent Original denture adhesive (available commercially manufactured by P&G), or for the following reference formula: Ca(47.5%)/Zn(17.5%) MVE/MA salt 33%, sodium carboxymethylcellulose 20%, mineral oil USP (65-75cst at 40C) 23.93%, petrolatum USP (consistency 17-20mm) 21.87%, colloidal silicon dioxide 1.14%, and Opatint OD 1646 0.06%; suitable methods to make this reference formula are disclosed in US 5,073,604, Holeva K., and US 6,617, 374 Rajaiah J.
(i) Calculate "Dislodgement Force Ratio" = (Peak Dislodgement Force of Prototype Adhesive) / (Peak Dislodgement Force of Fixodent Original)
Data: Each sample is repeated a minimum of 3 times and the average value of the "Normalized Dislodgement Force" and "Dislodgement Force Ratio" are reported.

Specifically the normalized dislodgement force and dislodgement force ratio can be measured by using the following parameters in the procedure: 0.25 gram adhesive; 1 inch diameter probe; hydration time of 20 minutes; and compression force of 7500 grams.

The "normalized ooze amount" and "ooze ratio" of the inventive article can be measured by the following procedure:
a. Load initial sample weight of about 0.50 grams uniformly onto a 1 inch diameter cylindrical probe made from polymethylmethacrylate,
b. Bring probe to 1.2 mm of base plate, also made from polymethylmethacrylate,
c. Apply 750 gram force for 90 seconds,
d. At 90 seconds, trim and weigh material that has oozed out,
e. Calculate "Normalized Ooze Amount" = (Amount oozed out / Initial sample weight) x 100,
f. Repeat Steps a-e using commercial Fixodent Original a denture adhesive cream commercially manufactured by P&G, or with the following reference formula: Ca(47.5%)/Zn(17.5%) MVE/MA salt 33%, sodium carboxymethylcellulose 20%, mineral oil USP (65-75cst at 40C) 23.93%, petrolatum USP (consistency 17-20mm) 21.87%, colloidal silicon dioxide 1.14%, and Opatint OD1646 0.06%; suitable methods to make this reference formula are disclosed in US 5,073,604, Holeva K., and US 6,617, 374 Rajaiah J.,
g. Calculate "Ooze Ratio" = Normalized Ooze Amount of Prototype Adhesive / Normalized Ooze Amount of Fixodent Original,
h. Each sample is repeated a minimum of 3 times and the average value of the "Normalized Ooze Amount" and "Ooze Ratio" are reported.

## Claims

1. A denture adhesive article comprising:
a) a homogeneous, bioerodible mixture of from 10% to 90% by weight of the article of a denture adhesive component; and from 2% to 90% by weight of the article of a water insoluble thermoplastic component selected from polyethylene, microcrystalline wax, elastomers and mixture thereof; and
b) at least one non-adhesive self supporting substrate;
wherein the article has dry tack.

2. The article of Claim 1 wherein the article is non-aqueous.

3. The article of Claim 1 wherein the water insoluble thermoplastic component is microcrystalline wax having a melting point of from 70°C to 90°C.

4. The article of Claim 1 wherein the article is flexible.

5. The article of Claim 1 wherein the denture adhesive component is at a level of from 10% to 90% by weight of the article and is selected from the group consisting of a salt or mixed salt of AVE/MA, the salt containing a cationic salt function comprising a cation selected from the group consisting of Group IA and Group 2A cations of the periodic table, yttrium, titanium, zirconium, vanadium, chromium, manganese, iron, nickel, copper, zinc, boron, aluminum, and mixtures thereof.

6. The article of Claim 1 wherein the denture adhesive component is a salt or mixed salt of AVE/MA, preferably wherein the cation is selected from the group consisting of strontium, zinc, iron, magnesium, calcium, sodium, and mixtures thereof, even more preferably wherein the salt is selected from the group consisting of a calcium/zinc salt of AVE/MA, a magnesium/zinc/sodium salt of AVE/MA, a calcium/sodium salt of AVE/MA, a zinc salt of AVE/MA and mixtures thereof.

7. The article of Claim 1 wherein the denture adhesive component comprises, at a level of from 5% to 60% by weight, a cellulose derivative selected from the group consisting of hydroxyethylcellulose, hydroxypropylmethylcellulose, carboxymethylcellulose, corn starch, and mixtures thereof, preferably wherein the cellulose derivative is sodium carboxymehtylcellulose.

8. The article of Claim 1 wherein the non-adhesive self supporting substrate is selected from the group consisting of polyester, polypropylene, nylon, rayon, cellulose acetate, non-adhesive cellulose derivatives, cellulose acetate, cloth, fibrous fleece, paper, plastic, leather, microcrystalline wax, synthetic fibers, natural fibers, and mixtures thereof, preferably wherein the non-adhesive self supporting substrate is in a physical form selected from the group consisting of sheets, films, non-woven, woven, continuous, chopped, foam, and combinations thereof.

9. The article of Claim 8 wherein the non-adhesive self supporting substrate is formed by a process selected from the group consisting of extrusion, casting, calendaring, coating, un-bonded, spraybonded, spun-bonded, needle-punched, carded, thermal bonded, hydroentangled, meltblown, aperture print bonded, needled, wet-laid, dry-laid, and combinations thereof.

10. The article of Claim 1 wherein the article further comprises a release liner.

11. The article of Claim 1 wherein the self supporting substrate is not bio-erodible.

## Patentansprüche

1. Zahnprothesenhaftmittelgegenstand, umfassend:
a) eine homogene, biologisch abbaubare Mischung aus: zu 10 % bis 90 %, bezogen auf das Gewicht des Artikels, eine Zahnprothesenhaftmittelkomponente und zu 2 % bis 90 %, bezogen auf das Gewicht des Artikels, eine in Wasser unlösliche thermoplastische Komponente, die ausgewählt ist aus Polyethylen, mikrokristallinem Wachs, Elastomeren und einer Mischung davon, und
b) mindestens ein nicht-haftendes, selbsttragendes Substrat,
wobei der Artikel eine Trockenklebrigkeit aufweist.

2. Artikel nach Anspruch 1, wobei der Artikel nicht wässrig ist.

3. Artikel nach Anspruch 1, wobei die in Wasser unlösliche thermoplastische Komponente ein mikrokristallines Wachs mit einem Schmelzpunkt von 70 °C bis 90 °C ist.

4. Artikel nach Anspruch 1, wobei der Artikel flexibel ist.

5. Artikel nach Anspruch 1, wobei die Zahnprothesenhaftmittelkomponente 10 Gew-% bis 90 Gew.-% des Artikels ausmacht und ausgewählt ist aus der Gruppe bestehend aus einem Salz oder einem gemischten Salz von AVE/MA, wobei das Salz eine kationische Salzfunktion umfasst, die ein Kation umfasst, das ausgewählt ist aus der Gruppe bestehend aus Kationen der Gruppe 1A und der Gruppe 2A des Periodensystems, Yttrium, Titan, Zirconium, Vanadium, Chrom, Mangan, Eisen, Nickel, Kupfer, Zink, Bor, Aluminium und Mischungen davon.

6. Artikel nach Anspruch 1, wobei die Zahnprothesenhaftmittelkomponente ein Salz oder ein gemischtes Salz von AVE/MA ist, wobei das Kation vorzugsweise ausgewählt ist aus der Gruppe bestehend aus Strontium, Zink, Eisen, Magnesium, Calcium, Natrium und deren Mischungen, wobei das Salz noch stärker bevorzugt ausgewählt ist aus der Gruppe bestehend aus einem Calcium/ Zink-Salz von AVE/MA, einem Magnesium/Zink/Natrium-Salz von AVE/MA, einem Calcium/Natrium-Salz von AVE/MA, einem Zinksalz von AVE/MA und deren Mischungen.

7. Artikel nach Anspruch 1, wobei die Zahnprothesenhaftmittelkomponente zu 5 Gew.-% bis 60 Gew.-% ein Cellulosederivat umfasst, das ausgewählt ist aus der Gruppe bestehend aus Hydroxyethylcellulose, Hydroxypropylmethylcellulose, Carboxymethylcellulose, Maisstärke und deren Mischungen, wobei das Cellulosederivat vorzugsweise Natriumcarboxymethylcellulose ist.

8. Artikel nach Anspruch 1, wobei das nicht-haftende, selbsttragende Substrat ausgewählt ist aus der Gruppe bestehend aus Polyester, Polypropylen, Nylon, Rayon, Celluloseacetat, nicht-haftenden Cellulosederivaten, Celluloseacetat, Tuch, Faservlies, Papier, Kunststoff, Leder, mikrokristallinem Wachs, synthetischen Fasern, natürlichen Fasern und deren Mischungen, wobei das nicht-haftende selbsttragende Substrat bevorzugt in einer physikalischen Form vorliegt, die ausgewählt ist aus der Gruppe bestehend aus Flächengebilden, Folien, Vlies, Gewebe, zusammenhängend, zerhackt, Schaumstoff und Kombinationen davon.

9. Artikel nach Anspruch 8, wobei das nicht-haftende, selbsttragende Substrat anhand eines Verfahrens ausgebildet wird, das ausgewählt ist aus der Gruppe bestehend aus Extrusion, Gießen, Kalandern, Beschichten, nicht gebunden, Spray-gebunden, in einem Spinnvliesverfahren gebunden, nadelgefilzt, kardiert, thermisch gebunden, wasserstrahlverfilzt, schmelzgeblasen, Blendendruck-gebunden, genadelt, nass gelegt, trocken gelegt und Kombinationen davon.

10. Artikel nach Anspruch 1, wobei der Artikel ferner eine Trennlage aufweist.

11. Artikel nach Anspruch 1, wobei das selbsttragende Substrat nicht biologisch abbaubar ist.

## Revendications

1. Article adhésif pour dentier comprenant :
a) un mélange homogène, bio-érodable de 10 % à 90 % en poids de l'article d'un composant adhésif pour dentier ; et de 2 % à 90 % en poids de l'article d'un composant thermoplastique insoluble dans l'eau choisi parmi le polyéthylène, la cire microcristalline, les élastomères et leur mélange ; et
b) au moins un substrat auto-portant non adhésif ;
dans lequel l'article a une adhésivité à sec.

2. Article selon la revendication 1, dans lequel l'article est non-aqueux.

3. Article selon la revendication 1, dans lequel le composant thermoplastique insoluble à l'eau est de la cire microcristalline ayant un point de fusion de 70 °C à 90 °C.

4. Article selon la revendication 1, dans lequel l'article est souple.

5. Article de la revendication 1, dans lequel le composant adhésif pour dentier est à un niveau de 10 % à 90 % en poids de l'article et est choisi dans le groupe constitué d'un sel ou d'un sel mélangé d'AVE/MA, le sel contenant une fonction de sel cationique comprenant un cation choisi dans le groupe constitué de cations du Groupe 1A et du Groupe 2A du tableau périodique, d'yttrium, de titane, de zirconium, de vanadium, de chrome, de manganèse, de fer, de nickel, de cuivre, de zinc, de bore, d'aluminium, et de leurs mélanges.

6. Article selon la revendication 1, dans lequel le composant adhésif pour dentier est un sel ou sel mélangé d'AVE/MA, de préférence dans lequel le cation est choisi dans le groupe constitué de strontium, de zinc, de fer, de magnésium, de calcium, de sodium, et de leurs mélanges, encore plus préférablement dans lequel le sel est choisi dans le groupe constitué d'un sel de calcium/zinc d'AVE/MA, d'un sel de magnésium/zinc/sodium d'AVE/MA, d'un sel de calcium/sodium d'AVE/MA, d'un sel de zinc d'AVE/MA et de leurs mélanges.

7. Article selon la revendication 1, dans lequel le composant adhésif pour dentier comprend, à un niveau de 5 % à 60 % en poids, un dérivé de cellulose choisi dans le groupe constitué d'hydroxyéthylcellulose, d'hydroxypropylméthylcellulose, de carboxyméthylcellulose, d'amidon de maïs, et de leurs mélanges, de préférence dans lequel le dérivé de cellulose est du carboxyméthylcellulose de sodium.

8. Article selon la revendication 1, dans lequel le substrat auto-portant non adhésif est choisi dans le groupe constitué de polyester, de polypropylène, de nylon, de rayonne, d'acétate de cellulose, de dérivés de cellulose non adhésifs, d'acétate de cellulose, de tissu, de non-tissé fibreux, de papier, de plastique, de cuir, de cire microcristalline, de fibres synthétiques, de fibres naturelles, et de leurs mélanges, de préférence dans lequel le substrat auto-portant non adhésif est sous une forme physique choisi dans le groupe constitué de feuilles, de films, de textiles non-tissés, tissés, continus, coupés, de mousse, et leurs combinaisons.

9. Article selon la revendication 8 dans lequel le substrat auto-portant non adhésif est formé par un procédé choisi dans le groupe constitué d'extrusion, de coulage, de calandrage, de revêtement, de non-lié, de lié par pulvérisation, de non-tissé, d'aiguilleté, de cardé, de thermo-lié, d'enchevêtré par voie hydraulique, de soufflé en fusion, de lié par ouverture imprimée, d'aiguillé, par procédé par voie humide, par voie sèche, et leurs combinaisons.

10. Article selon la revendication 1, dans lequel l'article comprend en outre une protection détachable.

11. Article selon la revendication 1, dans lequel le substrat auto-portant n'est pas bio-érodable.
